# EUROPÄISCHE PATENTSCHRIFT

(11) **EP 1 392 330 B1**
(45) Veröffentlichungstag und Bekanntmachung des Hinweises auf die Patenterteilung: **28.06.2006**
(21) Anmeldenummer: 02738085.6
(22) Anmeldetag: 17.05.2002
(51) Int. Cl.: A61K 31/715, A61P 29/00

(54) **VERWENDUNG VON LPS ZUR BEHANDLUNG INTESTINALER ENTZÜNDUNGSPROZESSE**
USE OF LPS FOR TREATING INTESTINAL INFLAMMATION PROCESSES
UTILISATION DE LPS POUR TRAITER DES PROCESSUS INFLAMMATOIRES INTESTINAUX

(30) Priorität: 23.05.2001 DE 10125179
(43) Veröffentlichungstag der Anmeldung: 03.03.2004
(73) Patentinhaber: Laves, Ralph-Georg, 30989 Gehrden (DE)
(72) Erfinder: GRIMMECKE, Hans-Dieter, 01454 Feldschlöschen (DE); KALOUSEK, Markus, Bruno, CH-6214 Chenkon (CH); SEIBOLD, Frank, CH-3122 Kehrsatz (CH)
(74) Vertreter: Schaeffer, Michael
(86) Internationale Anmeldenummer: PCT/EP2002/005450
(87) Internationale Veröffentlichungsnummer: WO 2002/094290

(56) Entgegenhaltungen:
- US-A- 5 935 938
- CHRISTIAN ALEXANDER UND ERNST TH. RIETSCHEL: "Bakterielle Lipopolysaccharide - Hochaktive Stimulatoren der angeborenen Immunität" BIOSPEKTRUM, Bd. 5, 1999, Seiten 275-281, XP001088732 in der Anmeldung erwähnt
- WIESE ANDRE ET AL: "The dual role of lipopolysaccharide as effector and target molecule." BIOLOGICAL CHEMISTRY, Bd. 380, Nr. 7-8, Juli 1999 (1999-07), Seiten 767-784, XP001084426 ISSN: 1431-6730 in der Anmeldung erwähnt
- REMBACKEN B J ET AL: "Non-pathogenic Escherichia coli versus mesalazine for the treatment of ulcerative colitis: a randomised trial" LANCET, XX, XX, Bd. 354, Nr. 9179, 21. August 1999 (1999-08-21), Seiten 635-639, XP004266787 ISSN: 0140-6736
- HODGSON H: "Prospects of new therapeutic approaches for Crohn's disease" LANCET, XX, XX, Bd. 353, Nr. 9151, 6. Februar 1999 (1999-02-06), Seiten 425-426, XP004265930 ISSN: 0140-6736

## Beschreibung

Die Erfindung betrifft die Verwendung von LPS zur Behandlung intestinaler Entzündungsprozesse. Hierbei werden im folgenden unter LPS natürlich vorkommende und synthetisch oder halbsynthetisch hergestellte Varianten verstanden.

Lipopolysaccharide (LPS) bestehen aus einer Lipidkomponente, dem Lipoid A, und einem kovalent an diese Membrandomäne gebundenen Polysaccharidteil. Die Polysaccharidregion setzt sich aus der terminalen O-spezifischen Kette, einer Substruktur aus bis zu 50 sich wiederholenden Oligosaccharideinheiten von meist zwei bis acht Monomeren, und der an das Lipoid A gebundenen Kemregion zusammen:
O-Kette - Kernregion - Lipoid A

Die O-spezifische Kette zeichnet sich durch extrem hohe Strukturvariabilität bei verschiedenen Spezies aus. Der Lipoid A-Teil ist verantwortlich für die unten beschriebene biologische Wirkung. Durch Variation des Acylierungsmusters des Lipoid A wird die biologische Wirkung moduliert. Diese reicht von der agonistischen Wirkung, wie sie in den meisten natürlich vorkommenden LPS-Varianten auftritt, (z.B. in Salmonella friedenau oder Salmonella abortus equi), bis zur antagonistischen Wirkung der LPS-Varianten pflanzensymbiontischer Mirkroorganismen oder synthetischer Derivate (C. Alexander und E.Th. Rietschel, Biospektrum 4,99 5 Jahrgang 1999, S. 275-281). Eine umfangreiche Darstellung der LPS findet sich auch bei Wiese,A.; Brandenburg,K.; Ulmer,A.J.; Seydel, U.; Muller-Leoennies,S.. The dual role of lipopolysaccharide as effector and target molecule. Biol. Chem. 380, S. 767-784.

Lipopolysaccharide sind hochpotente Stimulatoren der angeborenen Immunität, welche von gram-negativen Bakterien gebildet werden. Sie binden an TLR4-/CD14-Rezeptoren auf humanen mononukleären Zellen und induzieren die Bildung bzw. Sekretion
- proinflammatorischer Zytokine wie TNFα, MIF, IL-1β, IL-6, IL-8, IL12, IL-15 und IL-18
- verschiedener koloniestimulierender Faktoren
- verschiedener Lipidmediatoren und
- reduzierter Sauerstoffspezies.

Zudem bewirkt LPS eine schnell einsetzende, antikörperunabhängige Aktivierung der Komplementkaskade unter Freisetzung der Anäphylatoxine C5a und C3a. Humane T-Lymphozyten werden zur Proliferation und zur Produktion von IL-2 und IFN angeregt (Alexander and Rietschel, 1999). Im weiteren Verlauf der Entzündungsreaktion wird die Bildung von Akutphase-Proteinen induziert.
Über diese Mechanismen aktivieren Lipopolysaccharide die unspezifische Immunantwort, wodurch Mikroorganismen, Viren und Tumorzellen besser eliminiert werden können. Als Folge der LPS-induzierten Produktion von Zytokinen und Lipidmediatoren werden auch Phänomene wie Entzündung, Fieber und Sepsis induziert. Wegen der toxischen Wirkung von LPS bei der Sepsis werden agonistische LPS auch Endotoxine genannt.

Der Einsatz von LPS-Antagonisten zur Behandlung der Sepsis und von LPS-Agonisten zur Verstärkung der immunologischen Tumoreradikation ist Gegenstand verschiedener Publikationen (Grimminger et al., Internist 38, 541-552, 1997, ikawa et ai., j NateCancerlnst 14, 1195-1201,1954).

Entzündungen können als Folge der Gewebeschädigung bei mechanischer oder chemischer Reizung, als Reaktion auf Infekte sowie als Resultat immunologischer Fehlreaktionen (Autoimmunerkrankungen/ chronischentzündliche Erkrankungen) entstehen. Akute Entzündungen kommen durch Elimination der Auslöser, also lysierte Zellen bei Gewebsschädigung oder Erreger bei Infekten, zum Stillstand. Chronische Entzündungen treten dann auf, wenn das auslösende Ereignis nicht eliminiert werden kann oder sich die Immunantwort verselbständigt, sich also eine Autoimmunerkrankung mit Angriff auf körpereigene Antigene oder auf die ubiquitäre Darmflora entwickelt.
Zentrales Ereignis aller Entzündungsreaktionen ist der Einstrom von Leukozyten in einen Entzündungsherd. Die rekrutierten Leukozyten schütten proinflammatorische, permeabilitätssteigernde und toxische Substanzen aus oder lösen die Ausschüttung solcher Substanzen durch andere Zellen aus (Zytokine, Enzyme, Mediatoren, Prostaglandine, Sauerstoffradikale). Dies führt zur erhöhten Durchblutung sowie zur Aktivierung und zum erhöhten Durchsatz von Immunzellen, welche an der Elimination des Erregers und am Abtransport von Abbauprodukten beteiligt sind. Verschiedene Wachstumsfaktoren - darunter auch Zytokine - fördern die Proliferation von Gewebezellen und somit den Heilungsprozeß.
Im Verlauf des Entzündungsprozesses werden aber auch gesunde Zellen angegriffen, was zu Gewebsschädigungen führt, welche bei chronischen Entzündungen anhalten und eine Gewebedegeneration zur Folge haben [Gemsa und Resch, 1997]. Entzündungen der Darmschleimhaut führen zudem zur Penetration von Allergenen und Nahrungsmittelantigenen durch die Darmwand. Die Folge ist eine übersteigerte Immunantwort gegen diese Epitope. T-Lymphozyten, welche dabei aktiviert werden, zirkulieren im lymphatischen System und kehren in mukosale oder dermale Regionen zurück [Kantele et al., 1986], [Czerkinsky et al., 1987], [Wenneras et al., 1994], [Holmgren et al., 1989], [Sztein et al., 1994], [Kantele et al., 1999]. Wenn sie dort auf dieselben oder kreuzreagierende Epitope stossen, kann dies lokale oder systemische allergische Reaktionen auslösen wie Nahrungsmittelallergie mit Urtikaria, Asthma, allergische Rhinitis oder Neurodermitis. Somit besteht ein direkter Zusammenhang zwischen intestinaler Entzündung, erhöhter Permeabilität und allergischen Erkrankungen [Hollander, 1999], [Ma, 1997], [Fink, 2001], [Nekam, 1998a], [Nekam, 1998b], [Pena, 1998], [Salminen et al., 1996], [Majamaa and Isolauri, 1997], [Dupont et al., 1989], [Malin et al., 1996]. Wegen Einzelheiten wird auf die Literaturliste verwiesen. Deshalb ist die Elimination der intestinalen Entzündung für eine kausale Therapie von großer Wichtigkeit, wenn intestinale Entzündungsreaktionen an der Pathogenese allergischer Erkrankungen beteiligt sind.

Bei Infekten mit gram-negativen Bakterien sind Lipopolysaccharide wichtige Auslöser der akuten Entzündung. Deshalb wurde bisher angenommen, daß LPS Entzündungsprozesse verstärken sollten.

Völlig überraschend wurde jetzt festgestellt, daß LPS, aus Mikroorganismen extrahiert oder voll- oder halbsynthetisch hergestellt, hervorragende Mittel zur Behandlung intestinaler Entzündungsprozesse darstellen.

LPS kann aus allen gram-negativen Mikroorganismen extrahiert und synthetisch derivatisiert werden.

Die wirksame Dosis der Substanz liegt im Bereich 0,05 ng/kg/d bis ca. 100 µg/kg/d, die bevorzugte Gabe bei oraler Anwendung bei etwa 1 ng/kg/d - 10 µg/kg/d. Da sich LPS bei oraler Gabe ganz im Gegensatz zur parenteralen Gabe durch geringe Toxizität auszeichnet, ist eine große therapeutische Breite gegeben. Daher sind bei oraler Gabe auch bei höheren Dosierungen bisher keine Nebenwirkungen festgestellt worden.

Die Verwendung von LPS erfolgt vorzugsweise oral oder rektal, z.B. in Form von Lösungen, Pulver, Granulaten, Tabletten, Kapseln, Dragees, Suppositorien oder Klysmen. Das LPS kann auch dadurch an den Wirkort Schleimhaut gebracht werden, dass LPS-produzierende oder-überproduzierende, nicht-pathogene Darmbakterien oral verabreicht werden. Transdermale, topische oder parenterale Anwendungen sind aber auch denkbar, letztere jedoch mit einer deutlich eingeschränkten therapeutischen Breite.

Bei Anwendung als Lösung ist es naheliegend, ein dem Pepton ähnliches, wässriges Lösungsmittel mit amphiphilen, wasserlöslichen Substanzen wie Fettsäure- und Lipidderivaten als Grundlage zu wählen, da insbesondere durch wasserlösliche Substanzen mit lipophiler Komponente eine gute Mizellierung als Voraussetzung der Löslichkeit gegeben ist. Dasselbe ist bei festen Darreichungsformen zu beachten, um eine ausreichende Solubilisierung und Bioverfügbarkeit in der Darmschleimhaut zu gewährleisten. Die Herstellung der pharmazeutischen Darreichungsform erfolgt im übrigen in einer dem Fachmann geläufigen Weise und entspricht Standardverfahren.

Beim verwendeten LPS handelt es sich um ein agonistisches LPS aus *Escherichia coli,* vorzugsweise Stamm Laves 1931, welches eine dem Referenz-LPS von Salmonella friedenau vergleichbare Aktivität im Sinne einer Stimulation der TNFα-Synthese in Monozyten aufweist. Der genaue Mechanismus, der hinter der entzündungshemmenden Wirkung steht, ist noch nicht bekannt.

Da aber die pharmakologische Wirkung von LPS, wie aus der Literatur hervorgeht (Alexander et al, Wiese et al), vom Lipoid A-Anteil, insbesondere von dessen Acylierung abhängt und nicht von der O-Kette oder der Kernregion, kann davon ausgegangen werden, daß in gleicher Weise alle LPS mit identischem oder ähnlichem Lipoid A-Acylierungsmuster und entsprechender agonistischer pharmakologischer Wirkung wirksam sind.

Oral appliziertes LPS wirkt in erheblichem Umfang entzündungshemmend.

Bei Untersuchung von Mäusen, welche aufgrund eines immunologischen Defekts (IL-10 /- knockout) ausgeprägte intestinale Entzündungen entwickeln [MacDonald T.T., 1994], hemmte LPS die Enzündung, was sowohl klinisch als auch histologisch nachgewiesen werden konnte.

Bei Untersuchungen an Mäusen, welche aufgrund einer chemischen Noxe eine intestinale Entzündungsreaktion zeigen, hemmte LPS diese Entzündungsreaktion, was ebenfalls klinisch als auch histologisch nachgewiesen werden konnte.

Die Erfindung wird im folgenden anhand der Beispiele näher erläutert:

### Beispiel 1:

### Präparative Isolierung von LPS

LPS kann durch verschiedene Methoden aus gram-negativen Bakterien extrahiert werden. Eine bevorzugte Methode wird hier vorgestellt:
Zur Gewinnung der LPS-haltigen Wirkstoffraktion aus *E. coli* wird bevorzugt *E. coli* des Serotyps 02:K1:H6 (vorzugsweise Stamm Laves 1931) fünf Tage bei 37C bis zu einer Wachstumsdichte von 3 x 10⁸ koloniebildende Einheiten /ml auf einem Medium auf Fleischpeptonbasis bebrütet. Es können aber auch andere Nährmedien, andere gram-negative Mikroorganismen und andere Wachstumsdichten verwendet werden. Aus der gewachsenen Kultur wird in an sich bekannter Weise durch thermische Lyse und Mehrfachfiltration ein eiweiß- und zellfreier Extrakt gewonnen, welcher eingeengt, mit einer 1kD-Membran gegen Wasser dialysiert und lyophilisiert wird. Alternativ kann die thermolysierte Biomasse bei 3900 g während 20 Minuten zentrifugiert und der Überstand lyophilisiert werden. Nach ausführlicher Dialyse mit einer 1kD-Membran gegen Wasser wird das Retentat eingeengt und lyophilisiert.

Die vorgestellte Methode zur Isolierung der Lipopolysaccharide entspricht derjenigen von Westphal et al [Westphal et al., 1952]. Das Lyophilisat wird in einem Wasser-Phenol Gemisch (55%-45%) suspendiert und homogenisiert, die Phasen werden durch Zentrifugation bei 2000 g getrennt.

Die Wasserphase wird wie oben beschrieben gegen Wasser dialysiert, eingeengt und lyophilisiert.
Das Lyophilisat wird in einer Konzentration von 10-40mg/ml in Wasser gelöst und bei 55000 g 3 mal 22 Stunden ültrazentrifügiert. Durch Lyophilisation des Sediments wird LPS-L31 erhalten, welches noch weiter (z.B. durch Gelfiltration) gereinigt werden kann.

### Beispiel 2:

### Hemmung intestinaler Entzündungsreaktionen bei Autoimmunerkrankungen

Folgende Experimente werden unter Doppelblindbedingungen an Interleukin 10 -/- knockout Mäusen vorgenommen [MacDonald T.T., 1994]. Zwei Gruppen von je 5 vier Wochen alten Mäusen erhielten während 21 Tagen entweder Placebo (Rinderpepton) bzw. Placebo + 14ng/ml Lipopolysaccharid aus lysierten *Escherichia coli,* Stamm Laves 1931 (LPS-L31, Verum) zu trinken. Im Verlauf der Behandlung wurde der Gewichtsverlauf verfolgt. Die Gewichtszunahme war in der Verumgruppe signifikant größer als in der Placebogruppe. Zudem wurden in der Placebogruppe häufiger klinische Zeichen einer Kolitis, wie Anusrötung, weicher Stuhl und transanaler Blutabgang beobachtet.
Nach dreiwöchiger Behandlung wurde der Darm asserviert und der Kryptenindex bestimmt. Der Kryptenindex stellt ein Maß für die Degeneration bzw. Hyperplasie der Darmwand, und somit ein Maß für die Entzündung dar: je größer die Zahl, desto stärker die Entzündung. Verummäuse hatten einen Kryptenindex von 4,75, Placebomäuse einen von 8.0. Daraus folgt, daß die mit LPS-L31 behandelten Mäuse eine signifikant geringere Entzündung aufweisen als die Placebomäuse. Bei der IL-10 -/- knockout Maus handelt es sich um ein anerkanntes Modell chronischentzündlicher Darmerkrankungen des Menschen.
Die Resultate zeigen, daß LPS-L31 Entzündungen aufgrund immunologischer Fehlsteuerung (Autoimmunkrankheiten) vermindert.

### Beispiel 3:

### Hemmung intestinaler Entzündungsreaktionen als Folge noxischer Einwirkung

In einem weiteren Experiment erhielten 2 Gruppen von je 6 Balb-c Mäusen (Alter 2 Monate) unter Doppelblindbedingungen während 1 Woche Placebo (Rinderpepton) bzw. Placebo + 14ng/ml LPS-L31 (Verum) zu trinken. Nach einwöchiger Behandlung erhielten alle Mäuse während 5 Tagen 2.5% DSS, um durch chemische Noxe eine Entzündung hervorzurufen. Danach erfolgte eine erneute Behandlung mit Placebo oder Verum während 6 Tagen. Die Gewichtszunahme war in der Verumgruppe signifikant größer als in der Placebogruppe. Der Kryptenindex war in der Verumgruppe signifikant kleiner als in der Placebogruppe, d.h. LPS-L31 verringerte die intestinale Entzündung. Zudem wurden in der Placebogruppe häufiger klinische Zeichen einer Kolitis, wie Anusrötung, weicher Stuhl und transanaler Blutabgang beobachtet.

### Beispiel 4:

### Bestimmung der Wirkung von LPS auf die Bildung von TNFα

Zur Untersuchung der Stimulation der TNFα-Sekretion in mononukleären Zellen wird wie publiziert vorgegangen [Thomsen and Loppnow, 1995]: Mononukleäre Zellen werden aus dem Blut gesunder Spender mit Hilfe einer Gradientenzentrifugation über Ficoll® isoliert, gewaschen und in Nährmedium (RPMI) 1640) inkubiert. Die Zellen werden 24 Stunden mit LPS bzw. Kontrollösung inkubiert, geerntet, mit 2% fötalem Kälberserum versehen und bis zur Zytokinanalyse bei -20C aufbewahrt. Die Bestimmung von TNFα erfolgt mit kommerziellem ELISA (Enzyme Linked Immunosorbent Assay) mit anti-TNFα Antikörper. 24 Stunden nach Zugabe von 1µg/ml LPS-L31 wurde eine TNFα-Konzentration von 1851 pg/ml gemessen, 1µg/ml Referenz-LPS aus Salmonella friedenau induzierte im selbem Zeitraum 1976 pg/ml TNFα. Daraus folgt, dass LPS-L31 ein agonistisches LPS darstellt, welches in seiner Aktivität nicht von bekannten LPS-Formen abweicht. Somit kann erwartet werden, dass alle agonistischen LPS-Formen bei intestinalen Entzündungen wirksam sind. Insbesondere ist keine antagonistische Wirkung nötig, um die Wirksamkeit sicherzustellen, wie dies aus der bisher bekannten Literatur zu erwarten wäre (Alexander et al, Wiese et al).

### Literaturliste

Alexander C, Rietschel ET (1999) Bakterielle Lipopolysaccharide - Hochaktive Stumulatoren der angeboren Immunität. *Biospektrum* 5: 275-281
Czerkinsky C. Prince SJ. Michalek SM, Jackson S, Russell MW, Moldoveanu Z, McGhee JR, Mestecky J (1987) IgA antibody-producing cells in peripheral blood after antigen ingestion: evidence for a common mucosal immune system in humans. *Proc Natl Acad Sci USA* 84: 2449-2453
Dupont C. Barai.E-. Molkhou P. Raynaud F, Barbet JP. Dehennin L (1989) Food-induced alterations of intestinal permeability in children with cow's milk-sensitive enteropathy and atopic dermatitis. *J Pediatr Gastroenterol Nutr* 8: 459-465
Fink MP (2001) Leaky gut hypothesis: a historical perspective (editorial). *Crit Care Med* 18: 579-580
Gemsa D. Resch K (1997) Entzündung. In *Immunologie,* Gemsa D, Kalden JR, Resch K (eds) pp 135-158. Georg Thieme Verlag: Stuttgart
Grimminger F. Mayer K, Seeger W (1997) Gibt es eine gesicherte Immuntherapie bei der Sepsis? *Internist* 38: 541-552
Hullander D (1999) Intestinal permeability, leaky gut, and intestinal disorders. *Curr Gustroenterol Rep* 1: 410-416
Holmgren J. Fryklund J, Larsson H (1989) Gamma-interferon-mediated down-regulation of electrolyte secretion by intestinal epithelial cells: a local immune mechanism ? *Scand J Immunol* 30: 499-503
Ikawa M. Koeptli JB. Mudd SG, Niemann C (1954) An agent from *E.coli* causing hemorrhage and regression of an experimental mouse tumor. *J Natl Cancer Inst* 14: 1195-1201
Kantele A. Arvilommi H. Jokinen [(1986) Specific immunoglobulin-secreting human blood cells after peroral vaccination against salmonella typhi. *J Infect Dis* 153: 1126-1131
Kantete A. Zivny J. Häkkinen M. Elson CO. Mestecky J (1999) Differential homing commitments of antigen-specitic T cells after oral or parenteral immunization in humans. *J Immunol* 162: 5173-5177
Ma TY (1997) Intestinal epithelial barrier dysfunction in Crohn's disease (44099). *P S E B M* 214: 318-327
MacDonald T.T. (1994) Gastrointestinal inflammation. Inflammatory bowel disease in knockout mice. *Curr Biol* 4: 261-263
Majamaa H. Isolauri E (1997) Probiotics: a novel approach in the management of food allergy. *J Allergy Clin Immunol* 99: 179-185
Malln M. Isotauri E. Pikkarainen P, Karikoski R, Isolauri J (1996) Enhanced absorption of macromolecules - A secondary factor in Crohn's disease. *Dig Dis .Sci* 41: 1423 -1428
Nekam K (1998a) Management of food allergy. *Allergy* 53: 122-124
Nekam KL (1998b) Nutritional triggers in asthma. *Acta Microbiol Immunol Hung* 45: 113-117
Pena AS (1998) Food allergy, coeliac disease and chronic inflammatory bowel disease in man. *Vet QQ* 20: 49-52
Salminen S. Isolauri E, Salminen E (1996) Clinical uses of probiotics for stabilizing the gut mucosal barrier: Successful strains and future challenges. *Int J Microbiol* 70:347-358
Sztein MB. Wasserman SS, Tacket CO, Edelman R. Hone D, Lindberg AA. Levine MM (1994) Cytokine production patterns and lymphoproliferative responses in volunteers orally immunized with attenuated vaccine strains of Salmonella typhi. *J Infect Dis* 170: 1508-1517
Thomsen A. Loppnow H (1995) Cytokine production by mononuclear cells following stimulation with a peptide-containing, endotoxin-free *Escherichia coli* extract. *Arzneim - Forsch*/*Drug Res* 45: 657-661
Wenneras C. Svennerholm AM. Czerkinsky C (1994) Vaccine-specific T cells in human peripheral blood after oral immunization with an inactivated enterotoxigenic Escherichia coli vaccine. *Inject Immun* 62: 874-879
Westphal O. Lüderitz O, Bister F.. (1952) Über die Extraktion von Bakterien mit Phenol/Wasser. Z *Naturforsch, B: Chem Sci* 7: 148-155

## Patentansprüche

1. Verwendung von LPS und/oder deren derivatisierten Varianten zur Herstellung eines Medikamentes zur peroralen oder rektalen Behandlung intestinaler Entzündungsprozesse.

2. Verwendung nach Anspruch 1, **dadurch gekennzeichnet, daß** ein agonistisches LPS eingesetzt wird.

3. Verwendung nach Anspruch 1 oder 2, **dadurch gekennzeichnet, daß** LPS aus E. coli eingesetzt wird.

4. Verwendung nach Anspruch 1 bis 3, **dadurch gekennzeichnet, daß** LPS aus E. coli des Serotyps O2:K1:H6 eingesetzt wird.

5. Verwendung nach Anspruch 1 bis 4, **dadurch gekennzeichnet, daß** LPS aus E. coli des Serotyps O2:K1:H6, Stamm Laves 1931 eingesetzt wird.

6. Verwendung nach Anspruch 1 bis 5, **dadurch gekennzeichnet, daß** die Dosierung bei oraler Gabe im Bereich von 0,05 ng/kg/d bis 100 µg/kg/d, vorzugsweise bei 1 ng/kg/d bis 10 µg/kg/d liegt.

7. Verwendung nach Anspruch 1 bis 6, **dadurch gekennzeichnet, daß** sie bei intestinalen Entzündungsprozessen aufgrund von Infektreaktionen erfolgt.

8. Verwendung nach Anspruch 1 bis 6, **dadurch gekennzeichnet, daß** sie bei intestinalen Entzündungsprozessen aufgrund von noxischen Einwirkungen, insbesondere chemischen Noxen erfolgt.

9. Verwendung nach Anspruch 1 bis 6, **dadurch gekennzeichnet, daß** sie bei intestinalen Entzündungsprozessen aufgrund von Autoimmunerkrankungen erfolgt.

10. Verwendung nach Anspruch 1 bis 6, **dadurch gekennzeichnet, daß** sie bei Colitiden verschiedenen Ursprungs oder bei allergischen Reaktionen aufgrund von intestinalen Entzündungen erfolgt.

## Claims

1. Use of LPS and/or derivatized variants thereof for the preparation of a medicament for peroral or rectal treatment of intestinal inflammation processes.

2. Use according to claim 1, **characterized in that** an agonistic LPS is employed.

3. Use according to claim 1 or 2, **characterized in that** LPS from E. coli is employed.

4. Use according to claim 1 to 3, **characterized in that** LPS from E. coli of serotype O2:K1:H6 is employed.

5. Use according to claim 1 to 4, **characterized in that** LPS from E. coli of serotype O2:K1:H6, strain Laves 1931 is employed.

6. Use according to claim 1 to 5, **characterized in that** the dosage for oral administration is in the range of from 0.05 ng/kg/d to 100 µg/kg/d, preferably 1 ng/kg/d to 10 µg/kg/d.

7. Use according to claim 1 to 6, **characterized in that** it takes place in cases of intestinal inflammation processes due to infection reactions.

8. Use according to claim 1 to 6, **characterized in that** it takes place in cases of intestinal inflammation processes due to exposure to noxae, in particular chemical noxae.

9. Use according to claim 1 to 6, **characterized in that** it takes place in cases of intestinal inflammation processes due to autoimmune diseases.

10. Use according to claim 1 to 6, **characterized in that** it takes place in cases of colitis of varying origin or in cases of allergic reactions due to intestinal inflammations

## Revendications

1. Emploi de lipopolysaccharides (LPS) et/ou de dérivés de LPS en vue de la préparation d'un médicament destiné au traitement, par voie orale ou rectale, de processus inflammatoires intestinaux.

2. Emploi conforme à la revendication 1, **caractérisé en ce que** l'on emploie un LPS agoniste.

3. Emploi conforme à la revendication 1 ou 2, **caractérisé en ce que** l'on emploie un LPS provenant d'E. coli.

4. Emploi conforme à l'une des revendications 1 à 3, **caractérisé en ce que** l'on emploie un LPS provenant d'E. coli du sérotype O2:K1:H6.

5. Emploi conforme à l'une des revendications 1 à 4, **caractérisé en ce que** l'on emploie un LPS provenant d'E. coli du sérotype O2:K1:H6, souche Laves 1931.

6. Emploi conforme à l'une des revendications 1 à 5, **caractérisé en ce que**, pour une administration orale, le dosage se situe dans l'intervalle allant de 0,05 ng/kg/j à 100 µg/kg/j, et de préférence dans l'intervalle allant de 1 ng/kg/j à 10 µg/kg/j.

7. Emploi conforme à l'une des revendications 1 à 6, **caractérisé en ce qu'**on y a recours en cas de processus inflammatoires intestinaux résultant de réactions à des infections.

8. Emploi conforme à l'une des revendications 1 à 6, **caractérisé en ce qu'**on y a recours en cas de processus inflammatoires intestinaux résultant d'influences nocives, en particulier d'agents chimiques toxiques.

9. Emploi conforme à l'une des revendications 1 à 6, **caractérisé en ce qu'**on y a recours en cas de processus inflammatoires intestinaux résultant de maladies auto-immunes.

10. Emploi conforme à l'une des revendications 1 à 6, **caractérisé en ce qu'**on y a recours en cas de colites de diverses origines ou de réac-tions allergiques résultant d'inflammations intestinales.
